# EUROPÄISCHE PATENTSCHRIFT

(11) **EP 3 948 218 B1**
(45) Veröffentlichungstag und Bekanntmachung des Hinweises auf die Patenterteilung: **21.05.2025**
(21) Anmeldenummer: 20718573.7
(22) Anmeldetag: 30.03.2020
(51) Int. Cl.: G01N 11/00, G01N 33/38, G01N 11/02

(54) **MESSUNG DER KONSISTENZ VON FRISCHBETON**
MEASURING THE CONSISTENCY OF FRESH CONCRETE
MESURE DE LA CONSISTANCE DE BÉTON FRAIS

(30) Priorität: 03.04.2019 DE 102019108780
(43) Veröffentlichungstag der Anmeldung: 09.02.2022
(73) Patentinhaber: PERI SE, 89264 Weißenhorn (DE)
(72) Erfinder: BÄCHLE, Helmut, 89264 Weißenhorn (DE); STAVES, Henning, 89264 Weißenhorn (DE); BERNARD, Vanessa, 89264 Weißenhorn (DE); BAUMANN, Rüdiger, 89264 Weißenhorn (DE)
(74) Vertreter: Holzwarth-Rochford, Andreas
(86) Internationale Anmeldenummer: PCT/EP2020/058901
(87) Internationale Veröffentlichungsnummer: WO 2020/201186

(56) Entgegenhaltungen:
- WO-A1-2008/111724
- CN-U- 206 930 553
- DE-A1- 10 007 612
- DE-A1- 102007 012 554
- JP-A- 2016 035 424
- JP-A- H07 280 716
- JP-B2- 6 434 249
- JP-B2- 6 562 534
- KR-A- 20160 008 891
- BISCOPING MICHAELA ET AL: "Frischbeton Eigenschaften und Prüfungen", ZEMENT-MERKBLATT BETONTECHNIK B4, 1 March 2013 (2013-03-01), pages 1 - 8, XP055951393, Retrieved from the Internet <URL:https://www.beton.org/service/zement-merkblaetter> [retrieved on 20220815]
- ANONYMOUS: "Motorized Cement Flow Table UTCM-0663E", INTERNET CITATION, 1 January 2020 (2020-01-01), pages 1 - 24, XP009538828, Retrieved from the Internet <URL:https://irp-cdn.multiscreensite.com/6d8c7494/files/uploaded/UTEST%20Product%20Catalogue.pdf>
- ACI MATERIALS ET AL: "Flow Test: Particle-Level and Macroscale Analyses", ACI MATERIALS JOURNAL TECHNICAL PAPER ACI MATERIALS JOURNAL, 30 June 2017 (2017-06-30), XP055712519, Retrieved from the Internet <URL:https://pdfs.semanticscholar.org/adba/907dc1685a560c02d3a7b37f80c49e90d161.pdf> [retrieved on 20200707]

## Beschreibung

Die Erfindung betrifft die Prüfung der Konsistenz von auf einer Baustelle angeliefertem Frischbeton sowie Gegenmaßnahmen für den Fall, dass diese Konsistenz sich als für die vorgesehene Verwendung ungeeignet erweist.

### Bezugnahme auf verwandte Anmeldungen

Die vorliegende Anmeldung beansprucht die Priorität der deutschen Patentanmeldung Nr. 10 2019 108 780.5, eingereicht am 3. April 2019, die in vollem Umfang durch Bezugnahme in das vorliegende Dokument aufgenommen wird.

### Stand der Technik

Frischbeton wird auf Baustellen meistens nicht selbst hergestellt, sondern von externen Lieferanten zugekauft. Einer der wichtigsten Parameter, der hierbei spezifiziert wird, ist die Fließfähigkeit des Betons. Eine auf der Baustelle eintreffende Lieferung von Frischbeton wird in der Regel mit dem genormten Ausbreitversuch dahingehend geprüft, ob die Fließfähigkeit der Spezifikation entspricht. Dabei wird eine Testfläche mit einer vorgegebenen Menge des Frischbetons belegt und nach einem vorgegebenen Zeitprogramm erschüttert. Der Durchmesser des dabei auf der Testfläche entstandenen Betongebildes wird zwei Mal über Kreuz gemessen, und anhand des aus beiden Messungen gebildeten Mittelwerts wird der Frischbeton in eine von sechs sogenannten Ausbreitmaßklassen F1-F6 eingeteilt. Diesen Ausbreitmaßklassen entsprechen Konsistenzbereiche, die von "steif" bis "sehr fließfähig" reichen.

(Hyun-Ki Kim et al., "Flow Test: Particle-Level and Macroscale Analyses", ACI Materials Journal Mai-Juni 2017, Titel 104-M36, 323-327) offenbart ein Verfahren für den Ausbreitungstest von Beton, bei dem Frischbeton auf eine Testfläche aufgebracht, die Testfläche erschüttert und anschließend aus Bildern der Testfläche die Konsistenz des Frischbetons ausgewertet wird.

DE 100 07 612 A1 offenbart einen Tisch für den Ausbreitungstest, der die Ausbreitung des Frischbetons mit Sensoren erfasst.

CN 206 930 553 U offenbart ein automatisiertes Testgerät für die Durchführung des Ausbreitungstests an Frischbeton.

WO 2008/111 724 A1 offenbart ein System zur automatisierten Auswertung des Ausbreitungstests für Frischbeton durch optische Beobachtung des fließenden Frischbetons.

KR 2016 0008 891 A1 offenbart ein weiteres Testgerät für die Durchführung des Ausbreitungstests.

JP 6 434 249 B2 offenbart ein weiteres automatisiertes Gerät, das die Ausbreitung von Frischbeton auf einer Testplatte initiiert und anschließend durch optische Beobachtung auswertet.

### Aufgabe und Lösung

Es ist eine Aufgabe der vorliegenden Erfindung, die Messgenauigkeit bei der Messung der Konsistenz von Frischbeton zu verbessern und die Messung zugleich reproduzierbarer zu machen. Es ist eine weitere Aufgabe der vorliegenden Erfindung, Maßnahmen für den Fall zur Verfügung zu stellen, dass die Konsistenz einer Lieferung Frischbeton von der Spezifikation für die vorgesehene Verwendung abweicht.

Diese Aufgaben werden erfindungsgemäß gelöst durch ein Verfahren zur Bestimmung der Konsistenz von Frischbeton gemäß Hauptanspruch 1, durch ein Verfahren zur Steuerung der Fließfähigkeit und/oder der Verwendung von Frischbeton gemäß Nebenanspruch 5, sowie durch eine Vorrichtung zur Bestimmung der Konsistenz von Frischbeton gemäß Hauptanspruch 7. Weitere vorteilhafte Ausgestaltungen ergeben sich aus den darauf rückbezogenen Unteransprüchen.

### Offenbarung der Erfindung

Im Rahmen der Erfindung wurde ein Verfahren zur Bestimmung der Konsistenz von Frischbeton entwickelt. Bei diesem Verfahren wird eine vorgegebene Menge des Frischbetons auf eine Testfläche aufgebracht. Diese Testfläche kann beispielsweise ein genormter Ausbreittisch sein. Die vorgegebene Menge kann beispielsweise mit einer für den Ausbreitversuch genormten Kegelstumpfform aufgebracht werden.

Die Testfläche wird nach einem vorgegebenen Zeitprogramm erschüttert. Dieses Zeitprogramm kann beispielsweise dem des genormten Ausbreitversuchs entsprechen.

Die Belegung der Testfläche mit dem Frischbeton wird nun aus mindestens einer Abbildung der Testfläche, und/oder durch mindestens einen an die Testfläche angekoppelten Sensor, erfasst. Aus der Belegung wird eine Kenngröße für die Konsistenz des Frischbetons ausgewertet.

Es wurde erkannt, dass auf diese Weise eine deutlich exaktere Aussage über die Konsistenz des Frischbetons gewonnen werden kann als durch Erfassung und Mittelung lediglich zweier Durchmesser. Diese Art der Messung war seinerzeit daraufhin optimiert worden, mit einfachsten Mitteln und sogar ohne Taschenrechner auf Baustellen durchgeführt werden zu können.

Die nun durch den Einsatz von Technik gewinnbare exaktere Bestimmung der Kenngröße für die Konsistenz des Frischbetons steigert auch die Aussagekraft des Vergleichs zwischen dieser Kenngröße und einem vorgegebenen Sollwert. Bei Abweichungen kann sinnvoll quantifiziert werden, wie groß diese Abweichungen sind, so dass angemessene Gegenmaßnahmen ergriffen werden können. Beispielsweise kann es bei nicht zu großen Abweichungen möglich sein, den Frischbeton nachträglich zu behandeln, um seine Kenngröße für die Konsistenz näher an den Sollwert zu bringen. Dies kann beispielsweise vollautomatisch geschehen, indem ein Bediener aufgefordert wird, eine bestimmte Menge eines Fließmittels oder anderen Zusatzstoffs dem Frischbeton zuzugeben. Es kann aber auch beispielsweise ein diesbezügliches Ansteuersignal erzeugt und ein Dosierer oder eine andere Vorrichtung für die Zugabe des Zusatzstoffs mit diesem Ansteuersignal angesteuert werden.

In einer weiteren vorteilhaften Ausgestaltung wird während und/oder nach dem Erschüttern der zeitliche Fortschritt der Belegung der Testfläche mit dem Frischbeton aus mehreren Abbildungen der Testfläche, und/oder aus einer zeitlichen Abfolge von Messwerten des mindestens einen Sensors, erfasst. Diese in der aktuellen Norm des Ausbreitversuchs nicht vorgesehene zusätzliche Erfassung des Zeitverlaufs erhöht die Aussagekraft weiter. So lässt beispielsweise eine in Sprüngen fortschreitende Belegung der Testfläche auf andere physikalische Vorgänge im Frischbeton schließen als eine bei gleicher Erschütterung der Testfläche kontinuierlich fortschreitende Belegung.

Zum Erschüttern der Testfläche wird mindestens ein elektromechanischer, elektromagnetischer, pneumatischer und/oder hydraulischer Aktor angesteuert. Auch dies erhöht die Genauigkeit und Reproduzierbarkeit. Die bisherige Norm für den Ausbreitversuch sieht vor, dass die Testfläche manuell an einem Handgriff bis zu einem Anschlag angehoben und anschließend fallengelassen wird. Hierbei ist die Erschütterung, mit der die Testfläche beim Anheben in den Anschlag fällt, nicht bestimmbar und nicht reproduzierbar. Weiterhin wird der Handgriff nicht immer gleich schnell losgelassen, so dass auch die Erschütterung, mit der die Testfläche zurückfällt, mit einer Unsicherheit behaftet ist. Schließlich ist auch die zeitliche Abfolge reproduzierbarer, wenn die Erschütterung durch Aktoren getrieben wird. Die bisherige Norm für den Ausbreitversuch sah recht große zeitliche Toleranzen für das Zeitprogramm der Erschütterungen vor.

In einer weiteren vorteilhaften Ausgestaltung wird die Belegung der Testfläche mit dem Frischbeton mit einem oder mehreren Drucksensoren, mit einem oder mehreren optischen Sensoren, und/oder mit einem oder mehreren kapazitiven Sensoren, erfasst. Insbesondere lässt es sich so ortsaufgelöst erfassen, wenn sich der Frischbeton ausgehend vom Zentrum der Testfläche beim Erschüttern der Testfläche nicht isotrop auf der Testfläche ausbreitet. Eine solche anisotrope Ausbreitung kann auf eine Unregelmäßigkeit bei der Herstellung des Frischbetons hindeuten.

Die Pixel der Abbildung der Testfläche werden dahingehend klassifiziert, ob sie zur freien Testfläche oder zum darauf befindlichen Frischbeton gehören. Auf diese Weise lässt sich der Anteil der Testfläche, der von dem Frischbeton belegt ist, ermitteln. Dieser, beispielsweise prozentuale, Anteil kann als Kenngröße für die Konsistenz des Frischbetons gewertet werden.

In Kombination hierzu kann beispielsweise ein parametrisierter Ansatz für die Form des auf der Testfläche befindlichen Frischbetons so an die Abbildung der Testfläche angepasst werden, dass Widersprüche mit dieser Abbildung minimiert werden. Auf diese Weise kann der Einfluss von Abbildungsfehlern reduziert werden.

In einer weiteren vorteilhaften Ausgestaltung wird die Abbildung der Testfläche perspektivisch zu einer senkrechten Aufsicht auf die Testfläche transformiert. Aus dieser Perspektive ist die Messung des vom Frischbeton bedeckten Flächenanteils besonders einfach. Ebenso lässt sich aus dieser Perspektive besonders einfach kontrollieren, ob sich der Frischbeton auf der Testfläche isotrop ausbreitet.

In einer weiteren besonders vorteilhaften Ausgestaltung wird die Testfläche vor dem Aufbringen des Frischbetons befeuchtet. Anhand mindestens einer Abbildung der Testfläche, und/oder mittels des Sensors, wird kontrolliert, dass die Beladung der Testfläche mit Wasser in einem vorgegebenen Soll-Bereich liegt.

Die bisherige Norm für den Ausbreitversuch sieht vor, dass die Testfläche befeuchtet und ein entstehender Wasserfilm anschließend abgezogen wird, um die Testfläche mattfeucht zu machen. Die Einhaltung dieser Randbedingung ist wichtig, damit das Ergebnis des Ausbreitversuchs nicht verfälscht wird. Hier wird nun eine quantitative Kontrolle eingeführt, was die Reproduzierbarkeit der gemessenen Kenngröße für die Konsistenz des Frischbetons nochmals verbessert.

In einer weiteren besonders vorteilhaften Ausgestaltung wird die Kenngröße für die Konsistenz des Frischbetons in Assoziation mit der Charge des Frischbetons protokolliert. "In Assoziation mit der Charge" bedeutet in diesem Zusammenhang, dass die Information der Charge zugeordnet werden kann, also beispielsweise unter einer Bezeichnung der Charge nachgeschlagen werden kann. Das Protokollieren kann beispielsweise in einer Datenbank auf einem Server, aber auch beispielsweise dezentral über eine Blockchain, erfolgen.

Die mit dem zuvor beschriebenen Verfahren verbesserte Messgenauigkeit bei der Bestimmung der Kenngröße für die Konsistenz macht diese Kenngröße nicht nur für die unmittelbare Verwendung auf der Baustelle aussagekräftiger, sondern auch für die Ursachenforschung in Fällen, in denen die Konsistenz von einem eigentlich gewünschten Wert abweicht. Wenn genügend Erfahrungen bezüglich der Konsistenzen verschiedener Chargen gesammelt werden, können hieraus, beispielsweise mittels maschinellem Lernen, Rückschlüsse gezogen werden, mit denen wiederum die Herstellung von Frischbeton besser gesteuert werden kann.

Besonders vorteilhaft werden zusätzlich Kenngrößen, die die stoffliche Zusammensetzung des Frischbetons charakterisieren, in Assoziation mit der Charge des Frischbetons protokolliert. Auf diese Weise kann beispielsweise geprüft werden, ob sich die Konsistenz des Frischbetons de facto reproduzierbar über die stoffliche Zusammensetzung steuern lässt oder ob diese Steuerung zumindest teilweise durch andere Einflüsse "überstimmt" wird.

Vorteilhaft wird zusätzlich ein durch die vorgesehene Verwendung des Frischbetons vorgegebener Sollwert der Kenngröße für die Konsistenz in Assoziation mit der Charge des Frischbetons protokolliert. Auf diese Weise ist eine noch genauere Analyse möglich, inwieweit sich beim Herstellungsprozess des Frischbetons ein vorgegebenes Ziel in Bezug auf die Konsistenz reproduzierbar ansteuern lässt. Insbesondere tritt zu Tage, ob sich beispielsweise eine Änderung der stofflichen Zusammensetzung immer so auswirkt wie dies beabsichtigt war.

Vorteilhaft werden weiterhin zusätzlich die Umgebungstemperatur, und/oder sonstige Witterungsbedingungen, in Assoziation mit der Charge des Frischbetons protokolliert. Diese sonstigen Witterungsbedingungen können beispielsweise einen Luftdruck und/oder eine Luftfeuchtigkeit umfassen. Auf diese Weise kann untersucht werden, inwieweit die Witterungsbedingungen die Konsistenz des Frischbetons beeinflussen. Hieraus lässt sich wiederum ableiten, in welcher Weise die Herstellung des Frischbetons den Witterungseinflüssen nachgeführt werden kann, um eine vorgegebene Konsistenz des Frischbetons zu erzielen.

Wenn ein sich über eine hinreichend große Anzahl von Chargen erstreckender Erfahrungsschatz protokolliert wurde, kann beispielsweise ein künstliches neuronales Netzwerk, KNN, daraufhin trainiert werden, aus der stofflichen Zusammensetzung des Frischbetons in Verbindung mit der gewünschten Kenngröße für die Konsistenz und den Witterungsbedingungen den sich tatsächlich einstellenden Wert dieser Kenngröße vorherzusagen. Dieses Training kann beispielsweise als überwachtes Training erfolgen. Das heißt, für jede im Erfahrungsschatz vorkommende Kombination aus stofflicher Zusammensetzung, Witterungsbedingungen und gewünschter Konsistenz wird mit dem KNN eine Vorhersage für die Konsistenz ermittelt und mit der tatsächlichen Konsistenz gemäß Erfahrungsschatz verglichen. Das KNN wird dann so angepasst, dass im Mittel die tatsächlich gemessenen Werte der Kenngröße für die Konsistenz reproduziert werden.

Die verbesserte Messgenauigkeit bei der Bestimmung der tatsächlichen Kenngröße für die Konsistenz bewirkt somit, dass das KNN präziser das sich tatsächlich einstellende Verhalten des Frischbetons vorhersagt. Dementsprechend wird auch die Verallgemeinerung der Vorhersage auf unbekannte Situationen, also auf unbekannte Kombinationen aus stofflicher Zusammensetzung, Witterungsbedingungen und gewünschter Konsistenz, besser funktionieren.

Ist das KNN erst einmal trainiert, so kann es genutzt werden, um die Herstellung des Frischbetons von vornherein so anzupassen, dass der Frischbeton mit einer höheren Wahrscheinlichkeit genau die beabsichtigte Konsistenz haben wird.

Beispielsweise kann das KNN aus dem besagten Erfahrungsschatz lernen, dass eine Kombination aus hoher Temperatur und geringer Luftfeuchtigkeit eine Tendenz hat, den Frischbeton zähflüssiger zu machen, und dass dieser Tendenz wiederum durch gleichzeitige Zugabe von Wasser sowie einem weiteren Zuschlagstoff entgegengewirkt werden kann.

Wie zuvor erläutert, wird mit der Verbesserung der Messgenauigkeit bei der Bestimmung der Kenngröße für die Konsistenz des Frischbetons insbesondere das Ziel verfolgt, in gewissem Umfang Gegenmaßnahmen zu ermöglichen und so eine nicht ganz der Spezifikation entsprechende Lieferung Frischbeton möglichst noch zu "retten", statt sie als Abfall entsorgen zu müssen.

Daher bezieht sich die Erfindung auch auf ein Verfahren zur Steuerung der Fließfähigkeit und/oder der Verwendung von Frischbeton.

Bei diesem Verfahren wird für eine Testmenge des Frischbetons eine Kenngröße für die Konsistenz mit dem zuvor beschriebenen Verfahren gemessen. Die gemessene Kenngröße wird mit einem für die vorgesehene Verwendung des Frischbetons vorgegebenen Sollwert verglichen.

Sofern die Abweichung vom Sollwert eine vorgegebene Toleranzschwelle noch nicht überschreitet, kann der Frischbeton noch ohne weiteres der vorgesehenen Verwendung zugeführt werden. Ist die Toleranzschwelle jedoch überschritten, wird geprüft, ob der Frischbeton derart behandelt werden kann, dass nach der Behandlung die Kenngröße nicht mehr um mehr als die Toleranzschwelle vom Sollwert abweicht.

Wenn diese Prüfung nun ergibt, dass die Abweichung durch die Behandlung unter die Toleranzschwelle gesenkt werden kann, dann wird die Behandlung durchgeführt. Mit der dann unterhalb der Toleranzschwelle liegenden Abweichung kann der Frischbeton doch noch seiner vorgesehenen Verwendung zugeführt werden. Hierbei empfiehlt es sich, die Kenngröße für die Konsistenz sicherheitshalber noch einmal wie zuvor beschrieben zu messen, um sicherzugehen, dass die Behandlung tatsächlich Erfolg hatte.

Ergibt die Prüfung hingegen, dass die Abweichung trotz Behandlung nicht unter die Toleranzschwelle gesenkt werden kann, wird der Frischbeton für die vorgesehene Verwendung gesperrt. Der Frischbeton kann möglicherweise durch die Behandlung noch für einen anderen Zweck auf der Baustelle tauglich gemacht werden. Alternativ kann er in unveränderter Form an den Lieferanten zurückgegeben werden.

Die Prüfung der Frage, ob die Abweichung durch die Behandlung unter die Toleranzschwelle gesenkt werden kann, kann insbesondere beispielsweise auch eine Prüfung beinhalten, ob der Einsatz des nachträglich behandelten Frischbetons für die vorgesehene Verwendung auf der Baustelle überhaupt zulässig ist. Wenn die Konsistenz des Frischbetons nicht der Spezifikation für die vorgesehene Verwendung entspricht, dann bedeutet dies letztendlich, dass die Zusammensetzung des Frischbetons nicht so ist, wie sie sein sollte. Das heißt, die abweichende Konsistenz ist mehr oder weniger ein Symptom des Grundübels, dass die Zusammensetzung nicht stimmt. Es gibt viele Möglichkeiten, die Konsistenz des Frischbetons im Nachhinein durch Behandlung anzupassen. Jedoch verleiht nicht jede solche Behandlung dem Frischbeton, bzw. dem hieraus später ausgehärteten Beton, sämtliche Eigenschaften, die auch ein von vornherein mit der richtigen Zusammensetzung gemischter Beton gehabt hätte. Daher kann es beispielsweise Vorgaben dahingehend geben, dass bestimmte, für die Standsicherheit des zu errichtenden Gebäudes besonders wichtige Teile nur aus Frischbeton hergestellt werden dürfen, der von vornherein ohne nachträgliche Behandlung die richtige Konsistenz hat. Diese Information kann beispielsweise einem dreidimensionalen Modell des zu errichtenden Gebäudes entnommen werden, das mit Informationen über die geplante Statik des Gebäudes angereichert ist.

In einer besonders vorteilhaften Ausgestaltung wird eine maximale Veränderbarkeit der Konsistenz des Frischbetons durch Behandlung unter Heranziehung der Rezeptur des Frischbetons ermittelt. Aus dieser Rezeptur ergibt sich insbesondere, inwieweit und in welche Richtung die Stöchiometrie des Frischbetons geändert werden kann, ohne dass er wichtige Eigenschaften verliert.

In einer besonders vorteilhaften Ausgestaltung wird eine Zugabe eines die Fließfähigkeit des Frischbetons erhöhenden Zusatzstoffs als Behandlung gewählt. Es kann also beispielsweise in Antwort darauf, dass die Testmenge des Frischbetons nicht fließfähig genug ist, ein Dosierer angesteuert werden, der der restlichen Menge des Frischbetons, oder einem Teil dieser Menge, den Zusatzstoff zuführt.

In einer weiteren vorteilhaften Ausgestaltung wird in Antwort darauf, dass der Frischbeton für die vorgesehene Verwendung gesperrt ist, geprüft, ob auf der gleichen Baustelle eine alternative Verwendung existiert, für die der Frischbeton angesichts seines aktuellen Werts der Kenngröße für die Konsistenz, und/oder angesichts eines ausgehend hiervon durch Behandlung erreichbaren Werts der Kenngröße, geeignet ist. In Antwort darauf, dass eine solche alternative Verwendung nicht, bzw. nicht in ausreichendem Maße (etwa nur für einen zu geringen Teil der angelieferten Menge), vorhanden ist, wird eine Ablehnung der Lieferung des Frischbetons ausgelöst. Diese Ablehnung kann sich dann beispielsweise darin manifestieren, dass das den Frischbeton anliefernde Fahrzeug zurück zum Lieferanten dirigiert wird.

In einer weiteren besonders vorteilhaften Ausgestaltung wird in Antwort darauf, dass der Frischbeton, ggfs. nach Behandlung, angesichts der Kenngröße für die Konsistenz für eine Verwendung (d.h., die ursprünglich vorgesehene oder eine alternativ aufgefundene Verwendung) geeignet ist, mindestens eine Fördereinrichtung für den Frischbeton dahingehend angesteuert, dass sie den Frischbeton dieser Verwendung zuführt. Diese Fördereinrichtung kann beispielsweise ein Betonschwenkarm sein. Es kann auch beispielsweise eine Abrechnung für den Frischbeton abhängig davon, für welche Verwendung der Frischbeton brauchbar ist, geändert werden. Die Information, welche Charge Frischbeton wo verwendet wurde, kann beispielsweise an ein Building Information Model, BIM, zurückgespielt werden. Das BMI ist ein "digitaler Zwilling" des zu errichtenden Gebäudes. Wird die Information, welcher Beton an welcher Stelle verwendet wurde, in das BMI zurückgespielt, kann hiermit beispielsweise später nachvollzogen werden, ob die Bauausführung im Hinblick etwa auf Standsicherheit und Dauerhaltbarkeit korrekt war. Wenn Mängel zu Tage treten, können beispielsweise Gebäudeteile einer besonderen Prüfung unterzogen bzw. turnusmäßige Prüfintervalle verkürzt werden.

Die beschriebenen Verfahren können ganz oder teilweise computerimplementiert ablaufen. Beispielsweise kann eine Abbildung der Testfläche mit einem Smartphone angefertigt und dort mit einer entsprechenden App auch direkt hinsichtlich der Ausbreitung des Frischbetons untersucht werden. Daher bezieht sich die Erfindung auch auf ein Computerprogramm mit maschinenlesbaren Anweisungen, die, wenn sie auf einem oder mehreren Computern und/oder Mobilgeräten ausgeführt werden, die Computer bzw. Mobilgeräte dazu veranlassen, eines der beschriebenen Verfahren auszuführen. Ebenso bezieht sich die Erfindung auch auf einen maschinenlesbaren Datenträger und/oder ein Downloadprodukt mit dem Computerprogramm.

Nach dem zuvor Beschriebenen bezieht sich die Erfindung auch auf eine Vorrichtung zur Bestimmung der Konsistenz von Frischbeton. Diese Vorrichtung umfasst eine Testfläche, auf der der Frischbeton aufbringbar ist. Die Testfläche ist so beschaffen, dass sich der Frischbeton bei Erschütterung der Testfläche auf der Testfläche ausbreitet. Es ist nun
- mindestens ein elektromechanischer, elektromagnetischer, pneumatischer und/oder hydraulischer Aktor für die Erschütterung der Testfläche und/oder
- mindestens ein an die Testfläche angekoppelter Sensor zur direkten oder indirekten Messung der Belegung der Testfläche mit dem Frischbeton
vorgesehen.

Wie zuvor erläutert, bewirken diese Maßnahmen einzeln oder in Kombination, dass die Bestimmung der Konsistenz deutlich reproduzierbarer wird und die erhaltenen Werte aussagekräftiger werden. Dies ist insbesondere dann vorteilhaft, wenn mittels Machine Learning oder anderen Analysetechniken weitergehende Schlüsse aus den Daten gezogen werden sollen.

Mindestens ein elektromechanischer, pneumatischer und/oder hydraulischer Aktor zum Anheben der Testfläche sowie mindestens ein elektromagnetischer Aktor zum ruckartigen Fallenlassen der Testfläche sind vorgesehen. Auf diese Weise kann die Testfläche insbesondere beispielsweise ruckfrei angehoben werden, so dass sie nur beim Fallenlassen in genau definierter Weise erschüttert wird.

In einer weiteren vorteilhaften Ausgestaltung ist mindestens ein Sensor als Drucksensor, und/oder als optischer Sensor, und/oder als kapazitiver Sensor, ausgebildet. Diese Sensoren sind insbesondere geeignet, um die Belegung der Testfläche mit dem Frischbeton ortsaufgelöst, sowie optional auch zeitaufgelöst, zu erfassen.

Die Vorrichtung kann insbesondere mit Schnittstellen für die Übergabe der erfassten Daten an eine Blockchain, ein Building Information Model, BIM, oder ein sonstiges Protokoll ausgerüstet sein.

### Spezieller Beschreibungsteil

Nachfolgend wird der Gegenstand der Erfindung anhand von Figuren erläutert, ohne dass der Gegenstand der Erfindung hierdurch beschränkt wird. Es ist gezeigt:
Figur 1: Ausführungsbeispiel des Verfahrens 100 zur Bestimmung der Konsistenz;
Figur 2: Beispielhafte Automatisierung des Erschütterns 120;
Figur 3: Beispielhafte sensorische Erkennung der Belegung 12 der Testfläche 1 mit dem Frischbeton 2;
Figur 4: Bestimmung der Belegung 12 der Testfläche 1 mit dem Frischbeton 2 aus einer Abbildung 1a der Testfläche 1;
Figur 5: Ausführungsbeispiel des Verfahrens 200 zur Steuerung der Fließfähigkeit und/oder der Verwendung von Frischbeton 2.

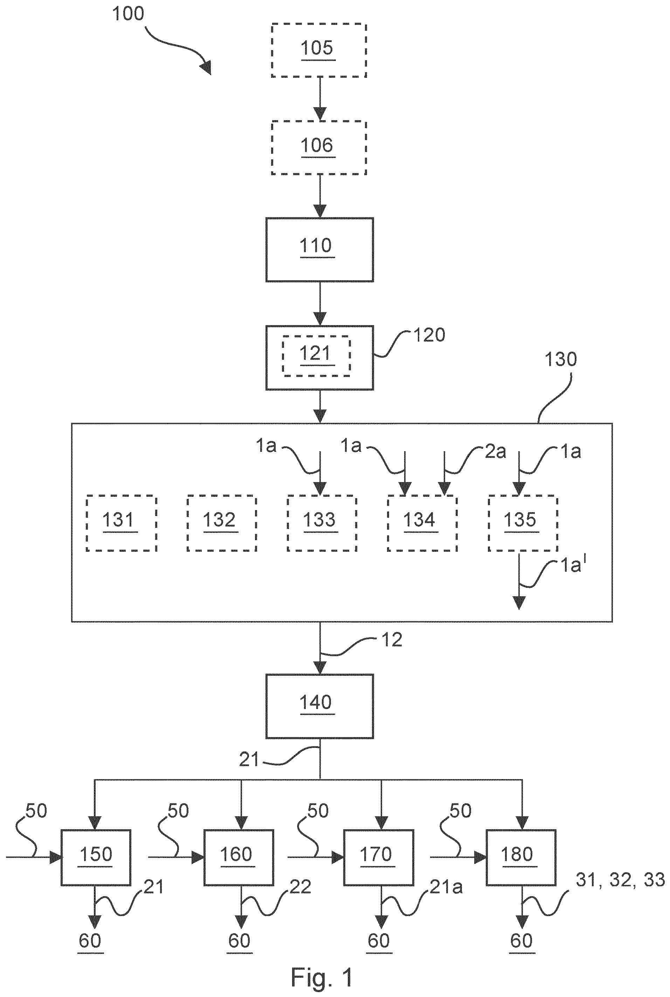

Figur 1 zeigt ein Ausführungsbeispiel des Verfahrens 100 zur Bestimmung einer Kenngröße 21 für die Konsistenz von Frischbeton 2. Im optionalen Schritt 105 wird eine Testfläche 1 zunächst befeuchtet, und im optionalen Schritt 106 wird kontrolliert, dass die Beladung der Testfläche 1 mit Wasser in einem vorgegebenen Soll-Bereich liegt.

In Schritt 110 wird eine vorgegebene Menge des Frischbetons 2 auf die Testfläche 1 aufgebracht. In Schritt 120 wird die Testfläche nach einem vorgegebenen Zeitprogramm erschüttert, wobei gemäß Block 121 hierfür ein oder mehrere elektromechanische, elektromagnetische, pneumatische und/oder hydraulische Aktoren 16a, 16b; 17a, 17b eingesetzt werden können.

In Schritt 130 wird die Belegung 12 der Testfläche mit dem Frischbeton 2 erfasst. Hierzu sind innerhalb des Kastens 130 verschiedene Möglichkeiten dargestellt.

Gemäß Block 131 kann nicht nur ein statischer Zustand der Belegung 12 erfasst werden, sondern auch der zeitliche Fortschritt dieser Belegung 12 verfolgt werden.

Gemäß Block 132 kann die Belegung 12 mit einem oder mehreren Drucksensoren, mit einem oder mehreren optischen Sensoren, und/oder mit einem oder mehreren kapazitiven Sensoren, erfasst werden. Es ist dann nicht nötig, eine Abbildung 1a der Testfläche 1 anzufertigen.

Gemäß Block 133 können bei der Ermittlung der Belegung 12 aus einer Abbildung 1a Pixel dieser Abbildung 1a dahingehend klassifiziert werden, ob sie zur freien Testfläche 1 oder zum darauf befindlichen Frischbeton gehören.

Gemäß Block 134 kann ein parametrisierter Ansatz 2a für die Form des auf der Testfläche 1 befindlichen Frischbetons 2 so an die Abbildung 1a der Testfläche 1 angepasst werden, dass Widersprüche mit dieser Abbildung 1a minimiert werden.

Gemäß Block 135 kann die Abbildung 1a der Testfläche 1 perspektivisch zu einer senkrechten Aufsicht 1a auf die Testfläche 1 transformiert werden. Damit kann insbesondere der Flächenanteil, der vom Frischbeton 2 auf der Testfläche 1 belegt ist, einfacher bestimmt werden.

Aus der Belegung 12 der Testfläche 1 mit dem Frischbeton 2 wird in Schritt 140 die Kenngröße 21 für die Konsistenz des Frischbetons 2 ausgewertet.

In Schritt 150 wird die so ermittelte Kenngröße 21 in Assoziation mit der Charge 50 des Frischbetons 2 in ein Protokoll 60 protokolliert.

In Schritt 160 werden zusätzlich Kenngrößen 22, die die stoffliche Zusammensetzung des Frischbetons 2 charakterisieren, in Assoziation mit der Charge 50 des Frischbetons 2 in das Protokoll 60 protokolliert.

In Schritt 170 wird zusätzlich ein durch die vorgesehene Verwendung des Frischbetons 2 vorgegebener Sollwert 21a der Kenngröße 21 in Assoziation mit der Charge 50 des Frischbetons 2 in das Protokoll 60 protokolliert.

In Schritt 180 werden zusätzlich die Umgebungstemperatur 31, und/oder sonstige Witterungsbedingungen (hier Luftdruck 32 und Luftfeuchtigkeit 33), in Assoziation mit der Charge 50 des Frischbetons 2 in das Protokoll 60 protokolliert.

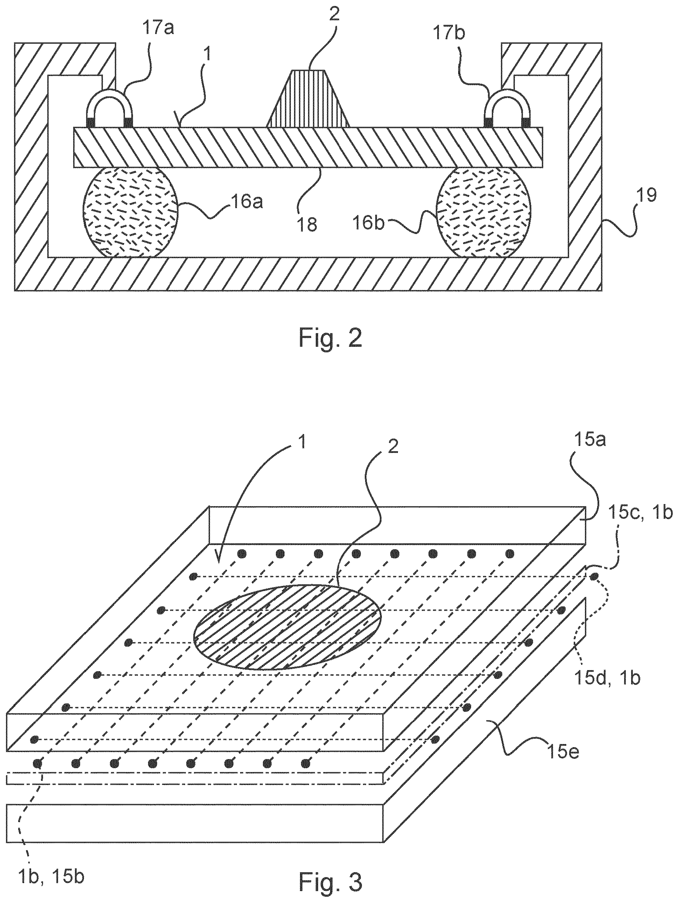

Figur 2 zeigt in nicht maßstabsgetreuer vereinfachter Schemazeichnung eine beispielhafte Möglichkeit, wie das Erschüttern 120 der Testfläche 1 automatisiert werden kann. Das Erschüttern wird hierdurch reproduzierbarer, so dass die Genauigkeit der letztendlich erhaltenen Kenngröße 21 für die Konsistenz weiter verbessert werden kann.

Die Testfläche 1 befindet sich auf der Oberseite einer beweglichen Platte 18, die relativ zu einem Grundkörper 19 bewegbar ist. Mit den pneumatischen Bälgen 16a und 16b lässt sich die bewegliche Platte 18, und somit auch die Testfläche 1 mit dem Frischbeton 2, ruckfrei anheben, bis sie an den beiden Elektromagneten 17a und 17b anschlägt. In dem in Figur 2 gezeigten Zustand ist der Frischbeton 2 gerade auf die Testfläche 1 aufgebracht worden, und es hat noch keine Erschütterung der Testfläche 1 stattgefunden.

Die Elektromagneten 17a und 17b werden bestromt und halten somit die bewegliche Platte 18. Zur Vorbereitung der Erschütterung der Testfläche 1 werden die pneumatischen Bälge 16a und 16b entlüftet, so dass sie den Fall der beweglichen Platte 18 nicht bremsen. Sodann werden die Elektromagnete 17a und 17b gleichzeitig ausgeschaltet. Die bewegliche Platte 18 fällt dann in genau definierter Weise auf den Grundkörper 19 zurück, so dass die Testfläche 1 mit dem Frischbeton 2 in reproduzierbarer Weise erschüttert wird und sich der Frischbeton 2 dementsprechend auf der Testfläche 1 ausbreitet.

Figur 3 zeigt in nicht maßstabsgetreuer vereinfachter Schemazeichnung eine Möglichkeit, die Belegung 12 der Testfläche 1 mit dem Frischbeton 2 durch einen Sensor 1b zu messen.

Die Testfläche 1 befindet sich auf der Oberseite einer nichtleitenden Platte 15a. Der Sensor 1b ist ein ortsaufgelöster kapazitiver Sensor und umfasst zwei gekreuzte Anordnungen 15b und 15d aus parallelen Drähten, die durch eine Kleberschicht 15c voneinander getrennt sind. Hieran grenzt eine weitere nichtleitende Platte 15e. Die nichtleitenden Platten 15a und 15e können beispielsweise aus Glas bestehen, aber auch aus für den Einsatz auf Baustellen robusteren nichtleitenden Materialien wie Plexiglas, Acrylglas oder Kunststoff.

Wenn sich Frischbeton 2 auf der Testfläche 1 befindet, ändert sich die gegenseitige Kapazität von Drähten, die sich an dieser Stelle unterhalb der Testfläche 1 kreuzen. Durch das Testen der gegenseitigen Kapazitäten sich kreuzender Drähte lässt sich somit ortsaufgelöst und ohne bewegliche Teile feststellen, welcher Anteil der Testfläche 1 von dem Frischbeton 2 belegt ist.

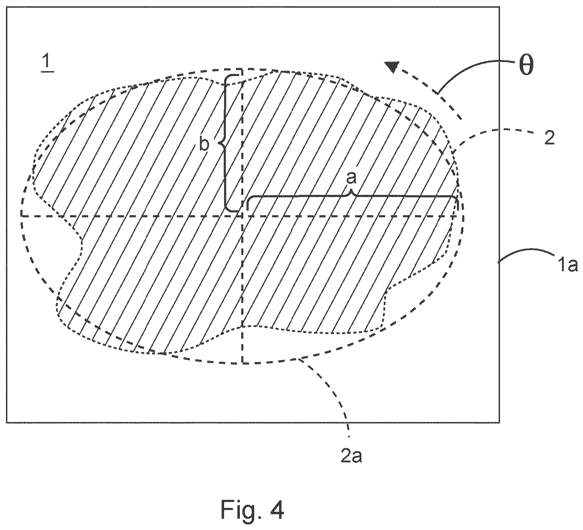

Figur 4 zeigt in nicht maßstabsgetreuer Schemazeichnung beispielhaft, wie die Belegung 12 der Testfläche 1 mit dem Frischbeton aus einer Abbildung 1a der Testfläche 11 gewonnen werden kann. Ein parametrisierter Ansatz 2a für die Form des auf der Testfläche 1 befindlichen Frischbetons wird durch Variation der Parameter so an die Abbildung 1a der Testfläche 1 angepasst, dass Widersprüche mit der Abbildung 1a minimiert werden.

In dem in Figur 4 gezeigten Beispiel beschreibt der parametrisierte Ansatz 2a eine Ellipse mit großer Halbachse a und kleiner Halbachse b, die noch um einen Winkel Θ gedreht sein kann.

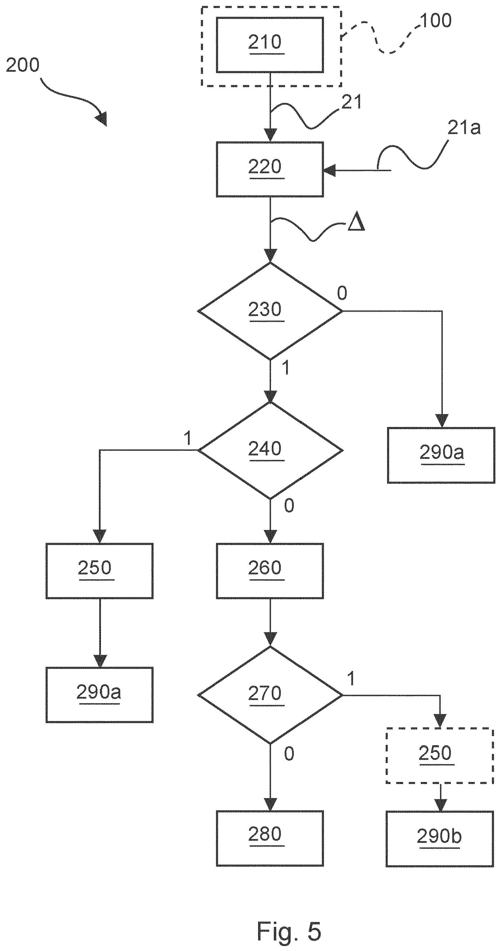

Figur 5 zeigt ein beispielhaftes Ablaufdiagramm des Verfahrens 200, mit dem die Fließfähigkeit und/oder die Verwendung von Frischbeton 2 gesteuert werden kann.

In Schritt 210 wird mit dem zuvor beschriebenen Verfahren 100 eine Kenngröße 21 für die Konsistenz des Frischbetons 2 gemessen. In Schritt 220 wird die gemessene Kenngröße 21 mit einem für die vorgesehene Verwendung des Frischbetons 2 vorgegebenen Sollwert 21a verglichen.

In Schritt 230 wird geprüft, ob die Abweichung Δ der gemessenen Kenngröße 21 vom Sollwert 21a eine Toleranzschwelle überschreitet. Ist dies nicht der Fall (Wahrheitswert 0), so kann in Schritt 290a eine Fördereinrichtung unmittelbar so angesteuert werden, dass der Frischbeton 2 seiner vorgesehenen Verwendung zugeführt wird.

Ist die Toleranzschwelle hingegen überschritten (Wahrheitswert 1 bei Schritt 230), wird in Schritt 240 geprüft, ob die Abweichung Δ durch eine Behandlung des Frischbetons 2 unter die Toleranzschwelle gesenkt werden kann. Ist dies der Fall (Wahrheitswert 1), wird in Schritt 250 die Behandlung des Frischbetons 2 durchgeführt. Anschließend wird in Schritt 290a die Fördereinrichtung so angesteuert, dass der Frischbeton 2 seiner vorgesehenen Verwendung zugeführt wird.

Kann hingegen die Abweichung Δ auch durch Behandlung des Frischbetons 2 nicht unter die Toleranzschwelle gesenkt werden (Wahrheitswert 0 bei Schritt 240), so wird in Schritt 260 der Frischbeton 2 für die beabsichtigte Verwendung gesperrt. Es wird anschließend in Schritt 270 geprüft, ob auf der gleichen Baustelle eine alternative Verwendung existiert, für die der Frischbeton 2 entweder in seinem aktuellen Zustand oder aber nach Behandlung verwendbar ist. Ist dies der Fall (Wahrheitswert 1), so wird, falls erforderlich, die Behandlung 250 durchgeführt. Anschließend wird in Schritt 290b die Fördereinrichtung so angesteuert, dass der Frischbeton 2 seiner alternativen Verwendung zugeführt wird.

Existiert hingegen keine alternative Verwendung (Wahrheitswert 0 bei Schritt 280), so wird eine Ablehnung der Lieferung des Frischbetons 2 ausgelöst.

### Bezugszeichenliste

- 1: Testfläche
- 1a: Abbildung der Testfläche 1
- 1a': senkrechte Aufsicht, generiert aus Abbildung 1a
- 1b: an die Testfläche 1 angekoppelter Sensor
- 12: Belegung der Testfläche mit Frischbeton 2
- 15a, 15e: Platten aus nichtleitendem Material
- 15b, 15d: Schichten paralleler Drähte, um 90° zueinander versetzt
- 15c: Kleberschicht zwischen Schichten 15b, 15d
- 16a, 16b: pneumatische Bälge, heben bewegliche Platte 18
- 17a, 17b: Elektromagnete, halten bewegliche Platte 18
- 18: bewegliche Platte mit Testfläche 1
- 19: Grundkörper
- 2: Frischbeton
- 2a: parametrisierter Ansatz für Form des Frischbetons 2
- 21: Kenngröße für Konsistenz des Frischbetons 2
- 21a: Sollwert für Kenngröße 21
- 22: Kenngrößen für stoffliche Zusammensetzung des Frischbetons 2
- 31: Temperatur
- 32: Luftdruck
- 33: Luftfeuchtigkeit
- 50: Charge des Frischbetons 2
- 60: Protokoll
- 100: Verfahren zur Bestimmung der Kenngröße 21 für die Konsistenz
- 105: Befeuchten der Testfläche 1
- 106: Prüfen der Beladung der Testfläche 1 mit Wasser
- 110: Aufbringen des Frischbetons 2 auf die Testfläche 1
- 120: Erschüttern der Testfläche 1
- 121: Erschüttern mittels Aktoren
- 130: Erfassen der Belegung 12 der Testfläche 1 mit Frischbeton 2
- 131: Erfassen des zeitlichen Fortschritts der Belegung 12
- 132: Erfassen der Belegung 12 mit Sensoren
- 133: Klassifizieren von Pixeln der Abbildung 1a
- 134: Anpassen des Ansatzes 2a an die Abbildung 1a
- 135: Transformieren der Abbildung 1a zur senkrechten Aufsicht 1a'
- 140: Auswerten der Kenngröße 21 aus der Belegung 12
- 150: Protokollieren der Kenngröße 21 für die Konsistenz
- 160: Protokollieren der Kenngrößen 22 für die stoffliche Zusammensetzung
- 170: Protokollieren des Sollwerts 21a für die Kenngröße 21
- 180: Protokollieren von Witterungseinflüssen 31, 32, 33
- 200: Verfahren zum Steuern der Fließfähigkeit und/oder Verwendung
- 210: Messen der Kenngröße 21
- 220: Vergleich der Kenngröße 21 mit Sollwert 21a
- 230: Prüfen, ob Abweichung Δ größer als Toleranzschwelle
- 240: Prüfen, ob Behandeln des Frischbetons 2 zielführend
- 250: Behandeln des Frischbetons 2
- 260: Sperren des Frischbetons 2 für beabsichtigte Verwendung
- 270: Prüfen auf alternative Verwendung für Frischbeton 2
- 280: Auslösen einer Ablehnung der Lieferung des Frischbetons 2
- 290a: Zuführen zur beabsichtigten Verwendung mit Fördereinrichtung
- 290b: Zuführen zur alternativen Verwendung mit Fördereinrichtung
- a: große Halbachse der Ellipse, die die Form 2a bildet
- b: kleine Halbachse der Ellipse
- Θ: Drehwinkel der Ellipse
- Δ: Abweichung

## Patentansprüche

1. Verfahren (100) zur Bestimmung der Konsistenz von Frischbeton (2) mit den Schritten:
• eine vorgegebene Menge des Frischbetons (2) wird auf eine Testfläche (1) aufgebracht (110);
• die Testfläche (1) wird nach einem vorgegebenen Zeitprogramm erschüttert (120), wobei die Testfläche (1) auf einer Oberseite einer beweglichen Platte (18) angeordnet ist, die relativ zu einem Grundkörper (19) bewegbar ist, und wobei die bewegliche Platte (18) mit der Testfläche (1) für das Erschüttern mittels mindestens eines elektromechanischen, pneumatischen und/oder hydraulischen Aktors (16a, 16b) vom Grundkörper (19) ruckfrei bis zu mindestens einem elektromagnetischen Aktor (17a, 17b) angehoben wird und mittels des mindestens einen elektromagnetischen Aktors (17a, 17b) ruckartig auf den Grundkörper (19) fallengelassen wird;
• die Belegung (12) der Testfläche (1) mit dem Frischbeton (2) wird aus mindestens einer Abbildung (1a) der Testfläche (1), sowie optional zusätzlich durch mindestens einen an die Testfläche (1) angekoppelten Sensor (1b), erfasst (130), und wobei optional während und/oder nach dem Erschüttern (120) der zeitliche Fortschritt der Belegung (12) der Testfläche (1) mit dem Frischbeton (2) aus mehreren Abbildungen (1a) der Testfläche (1), und/oder aus einer zeitlichen Abfolge von Messwerten des mindestens einen Sensors (1b), erfasst wird (131);
• aus der Belegung (12) wird eine Kenngröße (21) für die Konsistenz des Frischbetons (2) ausgewertet (140), wobei Pixel der Abbildung (1a) der Testfläche (1) dahingehend klassifiziert werden (133), ob sie zur freien Testfläche (1) oder zum darauf befindlichen Frischbeton (2) gehören und der Anteil der Testfläche (1), der von Frischbeton (2) belegt ist, als Kenngröße (21) des Frischbetons (2) gewertet wird.

2. Verfahren (100) nach Anspruch 1, wobei die Belegung (12) der Testfläche (1) mit dem Frischbeton (2) mit einem oder mehreren Drucksensoren, mit einem oder mehreren optischen Sensoren, und/oder mit einem oder mehreren kapazitiven Sensoren, erfasst wird (132).

3. Verfahren (100) nach einem der Ansprüche 1 bis 2, wobei die Abbildung (1a) der Testfläche (1) perspektivisch zu einer senkrechten Aufsicht (1a') auf die Testfläche (1) transformiert wird (135).

4. Verfahren (100) nach einem der Ansprüche 1 bis 3, wobei die Testfläche (1) vor dem Aufbringen (110) des Frischbetons (2) befeuchtet wird (105) und wobei anhand mindestens einer Abbildung (1a) der Testfläche (1), und/oder mittels des Sensors (1b), kontrolliert wird (106), dass die Beladung der Testfläche (1) mit Wasser in einem vorgegebenen Soll-Bereich liegt.

5. Verfahren (200) zur Steuerung der Fließfähigkeit und/oder der Verwendung von Frischbeton (2) mit den Schritten:
• für eine Testmenge des Frischbetons (2) wird eine Kenngröße (21) für die Konsistenz mit dem Verfahren (100) nach einem der Ansprüche 1 bis 4 gemessen (210);
• die gemessene Kenngröße (21) wird mit einem für die vorgesehene Verwendung des Frischbetons (2) vorgegebenen Sollwert (21a) verglichen (220);
• in Antwort auf die Feststellung (230), dass die Abweichung Δ der gemessenen Kenngröße (21) vom Sollwert (21a) eine Toleranzschwelle überschreitet, wird geprüft (240), ob der Frischbeton (2) derart behandelt werden kann, dass nach der Behandlung die Kenngröße (21) nicht mehr um mehr als die Toleranzschwelle vom Sollwert (21a) abweicht, wobei optional
o eine maximale Veränderbarkeit der Konsistenz des Frischbetons (2) durch Behandlung unter Heranziehung der Rezeptur des Frischbetons (2) ermittelt wird, und/oder
o eine Zugabe eines die Fließfähigkeit des Frischbetons (2) erhöhenden Zusatzstoffs als Behandlung gewählt wird;
• wenn die Prüfung (240) ergibt, dass die Abweichung Δ durch die Behandlung unter die Toleranzschwelle gesenkt werden kann, dann wird die Behandlung durchgeführt (250);
• wenn die Prüfung (240) ergibt, dass die Abweichung Δ trotz Behandlung nicht unter die Toleranzschwelle gesenkt werden kann, wird der Frischbeton (2) für die vorgesehene Verwendung gesperrt (260).

6. Verfahren (200) nach Anspruch 5, wobei
• in Antwort darauf, dass der Frischbeton (2) für die vorgesehene Verwendung gesperrt ist (260), eine Abrechnung für den Frischbeton (2) geändert wird, und/oder geprüft wird (270), ob auf der gleichen Baustelle eine alternative Verwendung existiert, für die der Frischbeton (2) angesichts seines aktuellen Werts der Kenngröße (21) für die Konsistenz, und/oder angesichts eines ausgehend hiervon durch Behandlung erreichbaren Werts der Kenngröße (21), geeignet ist, und wobei eine Ablehnung der Lieferung des Frischbetons (2) ausgelöst wird (280), wenn die alternative Verwendung nicht, bzw. nicht in ausreichendem Maße, vorhanden ist, und/oder
• in Antwort darauf, dass der Frischbeton (2), ggfs. nach Behandlung, angesichts der Kenngröße (21) für die Konsistenz für eine Verwendung geeignet ist, mindestens eine Fördereinrichtung für den Frischbeton (2) dahingehend angesteuert wird (290a, 290b), dass sie den Frischbeton (2) dieser Verwendung zuführt.

7. Vorrichtung zur Bestimmung der Konsistenz von Frischbeton (2), umfassend eine Testfläche (1), auf der der Frischbeton (2) aufbringbar ist, wobei die Testfläche (1) so beschaffen ist, dass sich der Frischbeton (2) bei Erschütterung der Testfläche (1) auf der Testfläche (1) ausbreitet, und wobei die Testfläche (1) auf einer Oberseite einer beweglichen Platte (18) angeordnet ist, die relativ zu einem Grundkörper (19) bewegbar ist, wobei
• mindestens ein elektromechanischer, elektromagnetischer, pneumatischer und/oder hydraulischer Aktor (16a, 16b; 17a, 17b) für die Erschütterung der Testfläche vorgesehen ist,
**dadurch gekennzeichnet, dass**
• mindestens ein elektromechanischer, pneumatischer und/oder hydraulischer Aktor (16a, 16b) zum Anheben der beweglichen Platte (18) mit der Testfläche (1) sowie mindestens ein elektromagnetischer Aktor (17a, 17b) zum ruckartigen Fallenlassen der beweglichen Platte (18) mit der Testfläche (1) auf den Grundkörper (19) vorgesehen sind.

8. Vorrichtung nach Anspruch 7, wobei zusätzlich mindestens ein an die Testfläche (1) angekoppelter Sensor (1b) zur direkten oder indirekten Messung der Belegung (12) der Testfläche (1) mit dem Frischbeton vorgesehen ist.

9. Vorrichtung nach Anspruch 8, wobei mindestens ein Sensor (1b) als Drucksensor, und/oder als optischer Sensor (1b), und/oder als kapazitiver Sensor (1b), ausgebildet ist.

## Claims

1. A method (100) for determining the consistency of fresh concrete (2), having the following steps:
• a predefined quantity of the fresh concrete (2) is applied (110) to a test area (1);
• the test area (1) is shaken (120) in accordance with a predefined time program, wherein the test area (1) is arranged on an upper side of a movable plate (18) which can be moved relative to a base body (19), and wherein the movable plate (18) with the test area (1) is raised smoothly from the base body (19) by means of at least one electromechanical, pneumatic and/or hydraulic actuator (16a, 16b) for the shaking up to at least one electromagnetic actuator (17a, 17b), and is dropped jerkily onto the base body (19) by means of the at least one electromagnetic actuator (17a, 17b);
• the coverage (12) of the test area (1) with the fresh concrete (2) is captured (130) from at least one image (1a) of the test area (1), as well as optionally additionally by at least one sensor (1b) coupled to the test area (1), and wherein optionally during and/or after the shaking (120), the temporal progress of the coverage (12) of the test area (1) with the fresh concrete (2) is captured (131) from multiple images (1a) of the test area (1) and/or from a temporal sequence of measured values of the at least one sensor (1b);
• a characteristic (21) for the consistency of the fresh concrete (2) is evaluated (140) from the coverage (12), wherein pixels of the image (1a) of the test area (1) are classified (133) as to whether they belong to the free test area (1) or to the fresh concrete (2) located thereon, and the proportion of the test area (1) which is covered by fresh concrete (2) is assessed as a characteristic (21) of the fresh concrete (2).

2. The method (100) according to Claim 1, wherein the coverage (12) of the test area (1) with the fresh concrete (2) is captured (132) with one or more pressure sensors, with one or more optical sensors, and/or with one or more capacitive sensors.

3. The method (100) according to any one of Claims 1 to 2, wherein the image (1a) of the test area (1) is transformed (135) in perspective to a vertical view (1a') of the test area (1).

4. The method (100) according to any one of Claims 1 to 3, wherein the test area (1) is moistened (105) prior to the application (110) of the fresh concrete (2) and wherein it is checked (106), based on at least one image (1a) of the test area (1) and/or by means of the sensor (1b), that the loading of the test area (1) with water lies in a predefined nominal range.

5. A method (200) for controlling the flowability and/or the use of fresh concrete (2) having the following steps:
• a characteristic (21) for the consistency for a test quantity of the fresh concrete (2) is measured (210) with the method (100) according to any one of Claims 1 to 4;
• the measured characteristic (21) is compared (220) with a predefined nominal value (21a) for the intended use of the fresh concrete (2);
• in response to the finding (230) that the deviation Δ of the measured characteristic (21) from the nominal value (21a) exceeds a tolerance threshold, it is checked (240) whether the fresh concrete (2) can be treated such that, following the treatment, the characteristic (21) no longer deviates from the nominal value (21a) by more than the tolerance threshold, wherein optionally
o a maximum variability of the consistency of the fresh concrete (2) is determined by treatment utilizing the formulation of the fresh concrete (2), and/or
o an addition of an additive which increases the flowability of the fresh concrete (2) is selected as the treatment;
• if the check (240) shows that the deviation Δ can be lowered below the tolerance threshold by the treatment, then the treatment is performed (250);
• if the check (240) shows that the deviation Δ cannot be lowered below the tolerance threshold despite treatment, the fresh concrete (2) is blocked (260) for the intended use.

6. The method (200) according to Claim 5, wherein
• in response to the fact that the fresh concrete (2) is blocked (260) for the intended use, the accounting for the fresh concrete (2) is amended, and/or it is checked (270) whether an alternative use exists on the same construction site, for which the fresh concrete (2) is suitable in view of its current value of the characteristic (21) for the consistency, and/or in view of a value of the characteristic (21) which can be achieved proceeding herefrom by treatment, and wherein a rejection of the delivery of the fresh concrete (2) is triggered (280) if the alternative use is not available or is not available to a sufficient extent, and/or
• in response to the fact that the fresh concrete (2), possibly after treatment, is suitable for a use in view of the characteristic (21) for the consistency, at least one conveying device for the fresh concrete (2) is actuated (290a, 290b) in that it feeds the fresh concrete (2) to this use.

7. A device for determining the consistency of fresh concrete (2), comprising a test area (1), to which the fresh concrete (2) can be applied, wherein the condition of the test area (1) is such that the fresh concrete (2) spreads on the test area (1) when the test area (1) is shaken, and wherein the test area (1) is arranged on an upper side of a movable plate (18) which can be moved relative to a base body (19), wherein
• at least one electromechanical, electromagnetic, pneumatic and/or hydraulic actuator (16a, 16b; 17a, 17b) is provided for shaking the test area,
**characterized in that**
• at least one electromechanical, pneumatic and/or hydraulic actuator (16a, 16b) is provided for raising the movable plate (18) with the test area (1), and at least one electromagnetic actuator (17a, 17b) is provided for jerkily dropping the movable plate (18) with the test area (1) onto the base body (19).

8. The device according to Claim 7, wherein at least one sensor (1b) is additionally provided that is coupled to the test area (1) for directly or indirectly measuring the coverage (12) of the test area (1) with the fresh concrete .

9. The device according to Claim 8, wherein at least one sensor (1b) is configured as a pressure sensor, and/or as an optical sensor (1b), and/or as a capacitive sensor (1b).

## Revendications

1. Procédé (100) destiné à déterminer la consistance de béton frais (2), comprenant les étapes :
• on applique (110) une quantité prédéfinie de béton frais (2) sur une surface d'essai (1) ;
• on met en vibration (120) la surface d'essai (1) selon un programme temporel prédéfini, la surface d'essai (1) étant disposée sur une face supérieure d'une plaque mobile (18) qui peut être déplacée par rapport à un corps de base (19) et la plaque mobile (18) avec la surface d'essai (1) étant soulevée pour la mise en vibration à l'aide d'au moins un actionneur électromécanique, pneumatique et/ou hydraulique (16a, 16b) depuis le corps de base (19) sans à-coups jusqu'à au moins un actionneur électromagnétique (17a, 17b) et à l'aide de l'au moins un actionneur électromagnétique (17a, 17b) étant relâchée par à-coups sur le corps de base (19) ;
• on détecte (130) le revêtement (12) de la surface d'essai (1) avec le béton frais (2) à partir d'au moins une image (1a) de la surface d'essai (1) ainsi que, en option, en plus par le biais d'au moins un capteur (1b) couplé à la surface d'essai (1), et dans lequel, en option pendant et/ou après la mise en vibration (120), on détecte (131) la progression dans le temps du revêtement (12) de la surface d'essai (1) avec le béton frais (2) à partir de plusieurs images (1a) de la surface d'essai (1), et/ou à partir d'une séquence temporelle de valeurs de mesure de l'au moins un capteur (1b) ;
• on évalue (140), à partir du revêtement (12), une grandeur caractéristique (21) pour la consistance du béton frais (2), des pixels de l'image (1a) de la surface d'essai (1) étant classés (133) à cet effet pour savoir s'ils appartiennent à la surface d'essai libre (1) ou au béton frais (2) se trouvant dessus et la part de la surface d'essai (1) qui est revêtue par le béton frais (2) est évaluée en tant que grandeur caractéristique (21) du béton frais (2).

2. Procédé (100) selon la revendication 1, dans lequel on détecte (132), avec un ou plusieurs capteurs de pression, avec un ou plusieurs capteurs optiques, et/ou avec un ou plusieurs capteurs capacitifs, le revêtement (12) de la surface d'essai (1) avec le béton frais (2).

3. Procédé (100) selon l'une quelconque des revendications 1 à 2, dans lequel on transforme (135) l'image (1a) de la surface d'essai (1) en perspective en une vue verticale (1a') sur la surface d'essai (1).

4. Procédé (100) selon l'une quelconque des revendications 1 à 3, dans lequel on humidifie (105) la surface d'essai (1) avant l'application (110) du béton frais (2), et dans lequel au moins une image (1a) de la surface d'essai (1), et/ou le capteur (1b), permet de contrôler (106) que la charge de la surface d'essai (1) en eau se trouve dans une plage de consigne prédéfinie.

5. Procédé (200) destiné à commander la coulabilité et/ou l'utilisation de béton frais (2), comprenant les étapes :
• on mesure (210) avec le procédé (100) selon l'une quelconque des revendications 1 à 4, pour une quantité d'essai de béton frais (2), une grandeur caractéristique (21) pour la consistance ;
• on compare (220) la grandeur caractéristique mesurée (21) à une valeur de consigne (21a) prédéfinie pour l'utilisation prévue du béton frais (2) ;
• en réponse à la constatation (230) que la variation Δ de la grandeur caractéristique mesurée (21) par rapport à la valeur de consigne (21a) dépasse un seuil de tolérance, on vérifie (240) si le béton frais (2) peut être traité de telle sorte que, après le traitement, la grandeur caractéristique (21) ne dévie plus de la valeur de consigne (21a) au-dessus du seuil de tolérance, dans lequel en plus
o on détermine une capacité de changement maximale de la consistance du béton frais (2) par un traitement en rapprochement de la recette du béton frais (2), et/ou
o on choisit d'ajouter un adjuvant améliorant la coulabilité du béton frais (2) pour le traitement ;
• lorsque la vérification (240) donne que la variation Δ peut descendre en-dessous du seuil de tolérance par le traitement, on réalise (250) alors le traitement ;
• lorsque la vérification (240) donne que la variation Δ ne peut pas descendre en-dessous du seuil de tolérance en dépit du traitement, on suspend (260) le béton frais (2) pour l'utilisation prévue.

6. Procédé (200) selon la revendication 5, dans lequel
• en réponse au fait que le béton frais (2) a été suspendu (260) pour l'utilisation prévue, une facturation pour le béton frais (2) est modifiée, et/ou on vérifie (270) s'il existe une utilisation alternative sur le même chantier pour laquelle le béton frais (2) est approprié au vu de sa valeur actuelle de la grandeur caractéristique (21) pour la consistance, et/ou au vu d'une valeur de la grandeur caractéristique (21) qui peut être atteinte à partir de celui-ci par l'intermédiaire du traitement, et dans lequel on déclenche (280) un rejet de la livraison du béton frais (2) lorsqu'il n'y a pas d'utilisation alternative, ou à un degré insuffisant, et/ou
• en réponse au fait que le béton frais (2) est approprié pour une utilisation, éventuellement après traitement, au vu de la grandeur caractéristique (21) pour la consistance, on commande (290a, 290b) à cet effet au moins un moyen de transport pour le béton frais (2) pour alimenter le béton frais (2) dans cette utilisation.

7. Dispositif destiné à déterminer la consistance de béton frais (2), comprenant une surface d'essai (1), sur laquelle le béton frais (2) peut être appliqué, dans lequel la surface d'essai (1) est conçue de telle sorte que le béton frais (2) s'étale sur la surface d'essai (1) lors de la mise en vibration de la surface d'essai (1), et dans lequel la surface d'essai (1) est disposée sur une face supérieure d'une plaque mobile (18) qui peut être déplacée par rapport à un corps de base (19), dans lequel
• au moins un actionneur électromécanique, électromagnétique, pneumatique et/ou hydraulique (16a, 16b ; 17a, 17b) est prévu pour la mise en vibration de la surface d'essai,
**caractérisé en ce qu'**
• au moins un actionneur électromécanique, pneumatique et/ou hydraulique (16a, 16b) est prévu pour soulever la plaque mobile (18) avec la surface d'essai (1) ainsi qu'au moins un actionneur électromagnétique (17a, 17b) pour relâcher par à-coups sur le corps de base (19) la plaque mobile (18) avec la surface d'essai (1).

8. Dispositif selon la revendication 7, dans lequel en plus au moins un capteur (1b) couplé à la surface d'essai (1) est prévu pour mesurer directement ou indirectement le revêtement (12) de la surface d'essai (1) avec le béton frais.

9. Dispositif selon la revendication 8, dans lequel au moins un capteur (1b) est configuré sous la forme d'un capteur de pression, et/ou sous la forme d'un capteur optique (1b), et/ou sous la forme d'un capteur capacitif (1b).
